# EUROPÄISCHE PATENTANMELDUNG

(11) **EP 3 025 761 A2**
(43) Veröffentlichungstag der Anmeldung: **01.06.2016**
(21) Anmeldenummer: 15002886.8
(22) Anmeldetag: 09.10.2015
(51) Int. Cl.: A61Q 19/00, A61Q 19/06, A61K 8/97, A61K 36/888, A23L 2/10, A23L 2/02, A23L 2/04, A61K 8/34

(54) **HERSTELLUNG EINES KOSMETISCHEN UND/ODER PHARMAZEUTISCHEN GRUNDSTOFFES AUS AQUATISCHEN PFLANZEN**

(30) Priorität: 11.10.2014 DE 102014015083
(71) Anmelder: Rogmans, Maria, 47546 Kalkar (DE)
(72) Erfinder: Hermann, Josef Wilhelm, 47546 Kalkar (DE)
(74) Vertreter: Schmidt, Karl Michael

(57) **Zusammenfassung**

Die Erfindung betrifft ein Verfahren zur Herstellung eines Grundstoffes aus aquatischen Pflanzen, gemäß Oberbegriff des Patentanspruches 1, sowie Herstellung eines kosmetischen oder dermatologischen Produktes oder Pflegeproduktes gemäß Oberbegriff des Patentanspruches 1, sowie kosmetisches oder dermatologisches Produkte selbst, sowie ein Kulturverfahren. Um hierbei, dass die in den Pflanzen enthaltenen wirkstoffe schonend behandelt werden, und das Extrakt eine höhere Ausgangskonzentration der Wirkstoffe aufweist, und die Wirkstoffe aus einer sortenreinen Kultur in reproduzierbarer Konzentration vorliegen, ist erfindungsgemäß vorgeschlagen, dass die geernteten aquatischen Pflanzen in ihrer Frischmasse ohne Zugabe von Wasser in einem Autoklaven geschlossen erhitzt werden.

## Beschreibung

Die Erfindung betrifft ein Verfahren zur Herstellung eines Grundstoffes aus aquatischen Pflanzen, gemäß Oberbegriff des Patentanspruches 1, sowie Herstellung eines kosmetischen oder dermatologischen Produktes oder Pflegeproduktes gemäß Oberbegriff des Patentanspruches 1, sowie kosmetisches oder dermatologisches Produkte selbst, sowie ein Kulturverfahren.

Aus der WO 2001/097771 A1 ist bereits der Einsatz von Extrakten der Pflanze der Gattung Pistia Stratiotes für Hautpflegemittel bekannt.

Die Pistia Stratiotes ist eine Aquakulturpflanze, und gehört, wie die Wasserlinse Lemnacea zur Familie der Araceae, d.h. der Aronstabgewächse.

Sie ist natürlich erheblich größer als die Wasserlinse und unterscheidet sich auch in ihrem Metabolismus von der Wasserlinse. Während die Lemnaceae eine merkliche Ausprägung als Schwachlichtpflanze haben, liegt bei der Pistia eher eine Normallicht-Ausprägung vor. Ebenso wie die Lemnacea vermehrt sich die Pistia weitestgehend vegetativ durch Sprossung, ist aber generell auch ein Blüher, der auch eine sexuelle Vermehrung über Samen generieren kann.

Der Zuwachs der Individuenzahl in einer Kulturanlage ist bei Lemnaceae aufgrund der erheblich höheren Anzahl von Individuen auf einer gegebenen Aquakulturfläche natürlich höher.

In Bezug auf einen erzielbaren Massenzuwachs zieht die Pistia aber nach, aufgrund ihres Vielfach größeren Pflanzencorpus.

Allgemein gilt die Pistia daher als stark populierend mit hohem Biomassenzuwachs, und wird in Süßwassergebieten daher sogar als invasiv eingestuft. Dies führt wiederum dazu, dass diese Pflanze zu unrecht geächtet wird.

In Bezug auf ihr Inhaltstoffspektrum ist die Pistia aber hoch wertvoll.

Diese können in Aquakulturanlagen kultiviert werden, wie sie aus der DE 10 2009 033 573 A1, sowie auch aus der WO 2010/089144 A1 bekannt sind.

Diese Systeme sind geschlossene Systeme mit einer hohen Raumdichte, so dass auf relativ kleinen Grundflächen ein Vielfaches der Biomasse davon in vertikalen Stapelkulturen erzeugt werden kann. Durch die Kultivierung in den genannten, hoch dichten Aquakulturanlagen, hat man die auf die freie Natur bezogene Invasivität völlig ausgeschaltet, und erhält stattdessen eine stark populierende Pflanze in einem geschlossenen System, mit hoher verfügbarkeit und hohem Biomassenertrag.

Der Erfindung liegt daher die Aufgabe zugrunde, ein Verfahren zur Herstellung eines Pflanzenextraktes aus aquatischen Pflanzen, sowie ein diesbezüglicher Grundstoff, sowie ein Kultivierverfahren zum Erhalt dieser dafür gewünschten Biomasse derart zu verbessern, dass die in den Pflanzen enthaltenen Wirkstoffe schonend behandelt werden, und der Extrakt eine höhere Ausgangskonzentration der Wirkstoffe aufweist, und die Wirkstoffe aus einer sortenreinen Kultur kommend in reproduzierbarer Konzentration vorliegen.

Die gestellte Aufgabe ist bei einem Verfahren der gattungsgemäßen Art erfindungsgemäß durch die kennzeichnenden Merkmale des Patentanspruches 1 gelöst.

weitere vorteilhafte Ausgestaltungen des Herstellverfahrens sind in den abhängigen Ansprüchen 2 bis 12 angegeben.

In. Bezug auf den Grundstoff ist die gestellte Aufgabe erfindungsgemäß durch die Merkmale des Patentanspruches 13 bis 16 gelöst.

Der Bezug auf eine dermatologische Verabreichung des Extraktes ist in Anspruch 17 angegeben.

In Bezug auf eine Kultivierverfahren ist die gestellte Aufgabe erfindungsgemäß durch die kennzeichnenden Merkmale des Patentes 18 gelöst.

Weitere vorteilhafte Ausgestaltungen des Kultivierverfahrens sind in den übrigen Ansprüchen angegeben 19 und 20.

Kern der Erfindung in Bezug auf die Herstellung des Grundstoffes ist, dass die geernteten aquatischen Pflanzen in ihrer Frischmasse ohne Zugabe von Wasser in einem Autoklaven geschlossen erhitzt werden.

Dabei kann das Verfahren auf folgende Pflanzen angewendet werden.
- Pistia stratiotes
- Eichhornia crassipes
- Azolla
- Lemnacea
- Spirodella
sowie alle Formen von Algen, die sich im Inhaltsstoffspektrum für einen konzentrierten Auszug nach dem angegebenen Verfahren eignen.

In Bezug auf ein bekanntes Verfahren zur Gewinnung der Inhaltsstoffe aus der Pistia Stratiotes ist aus der WO 2001/097771 A1 bekannt, die Pistia pro 300 Gramm getrocknete Pflanzenmasse mit 4,5 Litern destilliertem Wasser auf einer Temperatur von 80 bis 85°C eine Stunde lang zu brühen. Dies ist von Nachteil, weil mit der vorherigen Auftrocknung auch Oxidationsvorgänge eingeleitet werden, die das Inhaltsstoffspektrum nachteilig beeinflussen können.

Dem gegenüber wird bei der vorliegenden Erfindung ein völlig anderer Weg beschritten.

Aufgrund der Tatsache, dass bei der Herstellung von großen Mengen dieser Pflanze der eingangs genannte Kulturreaktor verwendet wird, liegt die Biomasse der Pistia sofort frisch vor, weil der Erntevorgang aus der Aquakulturanlage nunmehr mit zum Produktionsprozess gehört. Die Biomasse brauch nicht, wie im genannten Stand der Technik langwierig in der Natur gesammelt werden, wobei sie naturgemäß schon beim Transport schnell auftrocknet, sondern die Biomasse wird in-situ geerntet und sogleich in-situ weiterverarbeitet.

Auf diese weise werden die oben genannten Oxidationsvorgänge unterdrückt, und sofort die FRISCHMASSE dem Auszugsverfahren zugeführt.

Es wird somit eine Auftrocknung der Pflanzen bewusst vermieden. Außerdem wird der Wasserkörper des resultierenden Auszuges komplett durch den Wasserkörper des Zellwassers der Pflanze dargestellt.

wahlweise kann die geerntete Frischmasse auch unter Luftabschluss, d.h. vakuumverpackt transportiert, und ggfs auch ansiliert werden.

Eine weitere Möglichkeit des Erhaltes des pflanzeneigenen Zellwassers ist die Schockfrostung direkt nach der Ernte.

Die direkte geerntete Frischmasse mit Wurzel, oder die als Frischmasse vakuumverpackt transportierte Frischmasse wird dabei als Frischmasse samt der kompletten Wurzel oder als Mutterpflanze oder Tochterpflanze oder Stolone zerkleinert und OHNE Zugabe von Wasser in einem Autoklaven erhitzt. Die Stolone sind die Verbindungen zwischen Mutter- und Tochterpflanze.

vorteil dieser Maßnahme ist, dass durch die Verwendung von Frischmasse die Inhaltsstoffe möglichst unversehrt sind. Als Wassergrundlage, dient dabei ausschließlich das eigene Zellwasser der Pistia. Die Erhitzung sowie die Abkühlung erfolgt dabei vollständig in dem besagten Autoklaven, damit freiwerdende flüchtige ätherische Inhaltsstoffe mit dem Kondensat in den Auszug zurücktropfen.

Hierbei wird der Deckel des Autoklaven sogar mechanisch beaufschlagt, damit die Kondensattropfen durch Rütteln zurücktropfen.

Danach erfolgt nur eine Abfilterung der festen Bestandteile. Eine Verdünnung des Suds hat somit nicht stattgefunden, weil kein Wasser extern zugeführt wurde. Der Sud ist beim erfindungsgemäßen Verfahren damit erheblich konzentrierter, als der Sud gemäß der WO 2001/097771 A1. Ebenso sind Voroxidationen damit ausgeschlossen, da die Biomasse als Frischmasse verwendet wird.

Aus diesem Grund liegen die GESAMTEN Inhaltsstoffe der Pistia weitestgehend ursprünglich und unverdünnt vor.

Schlussendlich kommt beim erfindungsgemäßen Verfahren noch hinzu, dass anders als bei Wilakulturen, das Kulturwasser in den verwendeten Kulturreaktoren konditioniert werden kann, insbesondere durch Zugabe von Vitaminen, die den gesamten Inhaltsstoffaufbau in der Pflanze begünstigen. D.h. das Inhaltsstoffspektrum ist mit der angewandten Kulturmethode durch reproduzierbare Konditionierung des Kulturwassers einstellbar. Dies ist völlig anders als bei wild geernteten Pflanzen, die immer auf anderem Kulturwasser stehen können und folglich nicht konstante Inhaltsstoffspektren aufweisen.

Die Biomasse ist durch tägliche oder wöchentliche Partialbeerntung sofort und als Frischmasse verfügbar. D.h. Erntezyklen und Produktionszyklen können zeitlich direkt zusammengeschaltet werden.

In weiterer vorteilhafter Ausgestaltung ist angegeben, dass die aquatischen Pflanzen oder Teile davon zuvor gewaschen und zerkleinert werden, und dass sie in dem Autoklaven für eine Dauer von 10 bis 60 Minuten bei einer Temperatur T ≤ 100°C erhitzt werden.

In weiterer vorteilhafter Ausgestaltung ist angegeben, dass der Autoklav samt Inhalt im geschlossenen Zustand abgekühlt wird, und ein Rücktropfvorgang das Kondenswasser samt darin enthaltener ätherischer Bestandteile in den so erhaltenen Sud rückgeführt.

Weiterhin ist vorteilhaft ausgestaltet, dass der Sud filtiert, und die rückgehaltene Festmasse ausgepresst wird, derart dass Sud und Presswasser zusammengeführt, und die festen Bestandteile aus der weiteren Bearbeitung abgetrennt werden.

Weiterhin ist vorteilhaft ausgestaltet, dass dem Sud in einer Menge von ca. 25 Volumenprozent des Sudvolumens Alkohol zugegeben und damit vermischt wird.

Zur Kostenreduktion bei der Herstellung ist es von vorteil, dass der Alkohol vergällt ist.

Zur Bildung einer vorteilhaften Cremekonsistenz ist angegeben, dass dem Gemisch nun Methylcellulose und/oder eine salben- oder Cremebasis zugeführt wird.

In weiterer vorteilhafter Ausgestaltung ist angegeben, dass dem Gemisch Glycerin zugegeben wird, zur Vermeidung einer Schmierung oder Schuppung auf der Haut.

Für die gesteuerte und reproduzierbare Inhaltsstoffspektren wird das Kulturwasser der Pistia mit folgenden Düngezugaben genährt:
- DL-Milchsäure
- Vitamine insbesondere Vitamin B12 (beta Carotin)
- Thio-Milchsäure
- Kaliumhydrogencarbonat
- Ascorbinsäure
- Methanol
- NPK-Dünger
- Steinmehl feinstgemahlen (Nanopartikel) wurzelgängig,

### Ansonsten weitere Spurenelemente

Eine weitere mögliche Maßnahme bei der Kultivierung kann der Wurzelbeschnitt sein, zur Beschleunigung des Eintrittes der Wirkstoffe über das Kulturwasser.

Von besonderer Bedeutung ist die erfindungsgemäße Ausgestaltung, dass die Pistia aber auch die anderen oben genannten Pflanzen vegetativ vermehren. Bei der Pistia geschieht dies auch durch eine Art Sprossung. Die Tochterpflanze teilt sich dabei von der Mutterpflanze durch selbsttätige Sprossung ab, wie auch andere Pflanzen aus der Familie der Araceae, wie bspw die Lemnaceae. Maßgebend, und Teil der Erfindung ist die leichte Separierbarkeit der Pflanzenteile, nämlich Mutterpflanze, Tochterpflanze, sowie den zwischen Mutter und Tochterpflanze wachsenden Sproß selbst .

Spross und/oder Tocherpflanze enthalten dabei noch höhere Anteile von Wuchshormonen, die eine regenerierende Wirkung aufweisen. Diese können für die Herstellung von kosmetischen und/oder dermatologischen Produkten eine große Rolle spielen.

Zusätzlich zur äußerlichen Verabreichung der Wirkstoffe der aquatischen Pflanzen, insbesondere der Pistia stratiotes, auf der Haut, gibt es insbesondere in der Verwendung als Anti-Cellulitis-Mittel folgende Möglichkeiten.

Zusätzlich oder anstatt der von außen auf die Haut verabreichten Gabe der Wirkstoffe oder des Grundstoffes aus aquatischen Pflanzen, vorzugsweise aus der Pistia stratiotes, kann derselbe als Wasserauszug hergestellte Grundstoff unter die Oberhaut injiziert werden, durch einzelne Injektionsnadeln oder eine Mehrfachanordnung von Injektionsnadeln auf einem Stempel oder einer Walze. Dies hat den Vorteil, dass die hochwirksamen Wirkstoffe direkt in das von der Cellulitis betroffene Gewebe injiziert werden können, und nicht erst dort hindiffundieren müssen.

Außerdem ist auch vorgesehen, dass die aquatischen Pflanzen zur Gewinnung von Resveratrol herangezogen werden. Dieser Wirkstoff, der zu den Polyphenolen gehört, ist auch in aquatischen Pflanzen vorhanden, und kann auf vorteilhaft einfache Weise nach dem selben angegebenen Kulturverfahren in den aquatischen Pflanzen begünstigt aufgebaut werden.

Weiterhin ist angegeben, dass für das erfindungsgemäße Verfahren als aquatische Pflanzen
Pistia stratiotes und/oder
Lemnaceae und/oder
Alternanthera philoxeroides und/oder
Eichhornia crassipes und/oder Azolla und/oder
Limnathemum cristatium und/oder
Spirodela
eingesetzt werden.

In weiteren vorteilhaften Ausgestaltungen ist angegeben, dass die genannten Pflanzen, oder die Blätter der genannten Pflanzen oder die zerkleinerten Teile der genannten Pflanzen vor der Erhitzung mit Natriumhydrogencarbonat bestäubt werden.

Dies unterstützt den Auszug während der Erhitzung, so dass die Inhaltsstoffe noch effektiver in die austretetende Flüssigkeit hineindiffundieren.

Eine Reihe von wertvollen Inhaltsstoffen sind in den besagten Pflanzen zu finden.

Wichtig ist dabei die erfindungsgemäß schonende Auszugsmethode.

**Gibberline** (Gibberellan) sind Phytohormone und pflanzliche Wachstumshormone, chemisch handelt es sich um Diterpene, und kommen vermehrt in Rosettenpflanzen vor, zu denen die Pistia auch gehört.

Cytokinins sind Pflanzen-Wachstumshormone. Sie sind essentielle antioxidative Wachstumsfaktoren, die das Altern von pflanzlichen Gemüsesellen verzögern, wobei sie auf menschliche Zellen einen ähnlichen Effekt haben.

weiterhin regeneriert es epidermale Hautzellen und Keratinozyten.

Ferner fördert es die Fibroblasten Proliferation, sowie die Kollagen und Elastin Synthese und erhöht die Hyaluronsäure Synthese.

Weiterhin begünstigt es die Reparatur- und Heilungsfähigkeit der Haut - chirurgische Wunden (hypertrophisch (Hypertrophie) und Abrassionsbehandlungen.

Palmitinsäure ist ebenfalls ein wichtiger Bestandteil der Hautbarriere und des Hautsäuremantels, und sollte daher in der Creme oder Cremebasis enthalten sein.

Palmitinsäure ist auch in der Pistia stratiotes exlthalten.

Der Auswuchs der Pistia vom Stadium der Jungpflanze bis hin zur erwachsenen Pflanze dauert etwa 4 Wochen.

Durch zeitversetzte Impfung der Aquakulturwannen mit Jungpflanzen kann durch eine zeitversetzte Beerntung aber erreicht werden, dass die Wannen in chronologischer Abfolge zeitversetzt beerntet werden und dabei die gesamte Aquakultureinrichtung aber täglich oder nach beliebigem Bedarf für die Production der Produkte Biomasse zur Verfügung stellt.

Neben den nachfolgend noch genauer ausgeführten Wirkungen liegen auch bakteriozide und fungizide Wirkungen, bspw für die Anwendung bei Fußpilz oder Hautpilz, vor.

Ebenso ist die Wirkung im Einsatz bei Haarpflege, schuppenabbau, sowie Haarwuchsförderung ggfs unter Beimischung von Koffein vorgesehen.

In Bezug auf Grundstoffe aus aquatischen Pflanzen besteht der Kern der Erfindung darin, dass der Grundstoff für dermatologische oder pharmazeutiche Anwendung, hergestellt ist nach dem Verfahren nach einem der Ansprüche 1 bis 12 für Salben oder Cremes zur Behandlung von Akne, und/oder Neurodermitis, und/oder Cellulitis und/oder Fettabbau im subcutanen Gewebe einsetzbar ist.

Desweiteren kann der besagte Grundstoff Ausgangsbasis für jewede weitere pharmazeutische Anwendung sein, bei der der besagte Grundstoff zumindest nach einem der Ansprüche 1 bis 5 hergestellt wurde.

Gleiches gilt für einen Grundstoff nach einem der Ansprüche 1 bis 5, zur Herstellung eines humanen Nahrungsergänzungsmittels,

So wie für einen Grundstoff nach einem der Ansprüche 1 bis 5, zur Herstellung eines Tierfuttermittels oder Tierfutterergänzungsmittels.

In Bezug auf ein Kultivierverfahren zur Erzeugung dieser Biomasse für die o.g. Anwendung besteht der Kern der Erfindung darin, dass die Pistia stratiotis, die unter reduziertem Tageslicht bei einer Beleuchtungsstärke von kleiner oder gleich ca 50% der natürlichen Beleuchtungsstärke der jeweiligen Region liegt, in der sie kultiviert wird, derart, dass die sexuelle Vermehrung der Pistia stratiotis unterdrückt und die vegetative Vermehrung so begünstigt wird.

So werden genetische variationen vermieden und die Kultur kann durch Selbstklonung sortenrein und damit in ihrem Inhaltsstoffspektrum konstant gehalten werden.

Dasselbe kann natürlich auch auf die anderen genannten aquatischen Pflanzen angewendet werden.

In weiterer vorteilhafter Ausgestaltung ist angegeben, dass DL-Milchsäure, und/oder
Vitamine, insbesondere Vitamin B12, und/oder
Thio-Milchsäure, und/oder
Kaliumhydrogencarbonat, und/oder
Ascorbinsäure, und/oder
Methanol, und/oder
NPK-Dünger, und/oder
feinstgemahlenes Steinmehl, und/oder
Spurenelemente,
dem Kulturwasser zugegeben wird bzw. werden.

In weiterer vorteilhafter Ausgestaltung ist angegeben, dass unmittelbar vor Gabe der genannten zu zu gegebenden Stoffe die aquatischen Pflanzen einen Wurzelschnitt erhalten. Dadurch dringen die im Kulturwasser verabreichten Substanzen besonders leicht in den Pflanzenkörper ein. Die Biomasse lässt sich so auf den Anwendungsfall inhaltsstofflich designen.

Im weiteren sind Ausführungsbeispiele für die Anwendung des erfindungsgemäßen Extraktes oder des Grundstoffes angegeben.

### Zur Lipolyseaktivität des Pistia Extraktes an Adipocyten:

Als Lypolyse bezeichnet man den körpereigenen Abbau an Fetten, die in den Adipocyten,d.h. den Fettzellen als Reserven vorhanden sind. Durch Lipasen werden diese enzymatisch zu kleineren molekularen Bruchstücken, den Fettsäuren und dem Glycerin gespalten. Die freien Fettsäuren können dann von Muskelzellen zur Energiegewinnung genutzt werden.

Vorbekannte Versuche hierzu haben gezeigt, dass aus subcutanem Gewebe herausgelöste und isolierte Adipocyten mit einem Pistia Extrakt reagierten. Nach ca 90 Minuten und einer Temperatur von 37 °C wurden signifikant messbare Mengen an Glycerin photospektrometrisch nachgewiesen. Siehe hierzu "J.de Pharmacologie; 1981; 12; 219-224. Die Signifikanz wurde natürlich in mehreren Vergleichsmessungen nachgewiesen.

Diese Ergebnisse der Untersuchungen belegen, dass die Extrakte von der Konzentration abhängige Lipolyseaktivität bei humanen Adipocyten in vitro zeigen, da die prozentuale Erhöhung des freigesetzten Glycerins mit steigender Pflanzenextraktkonzentration ansteigt. Damit ist in generale die Wirksamkeit des Pistia stratiotes Extraktes als gewebefettabbauende Substanz auf der Haut und für die Anti-Cellulitis nachgewiesen.

Bei der hier zitierten Studie ist diese Aktivität aber eindeutig von der Konzentration des Substrates abhängig.

Mit diesem Anwendungsbeispiel werden nun auch die mit dem vorliegenden erfindungsgemäßen Verfahren zur Extraktion der Pflanze gegebenen Unterschiede zum Stand der Technik sehr deutlich.

So werden beim erfindungsgemäßen Verfahren die zerkleinerten Pflanzenbestandteile der Pistia stratiotes nicht zuvor getrocknet, sondern als Frischmasse erhitzt. Dies führt dazu, dass die Wirkstoffe nicht erst einer Oxydation zugeführt werden, sondern ursprünglich vorliegen. Weiterhin wird kein Wasser zugeführt, sondern die zerkleinerte Frischmasse direkt im Autoklaven unter Luftabschluss erhitzt. Als Flüssigkeit liegt in diesem Verfahrensschritt ausschließlich nur das Zellwasser der Pflanze vor.

Damit ist der resultierende Wasserkörper auch wesentlich kleiner, und damit ist der anschließend abgepresste oder abfiltrierte Saft erheblich konzentrierter.

Darüber hinaus kommt noch hinzu, dass in einer bevorzugten Verfahrensausgestaltung die Temperatur unter 80°C, ggfs sogar erheblich darunter gehalten wird, was bewirkt, dass der so durch Brühung erhaltene Auszug schonender behandelt wurde, was auch die Qualität des Auszuges begünstigt.

Alles in allem liegt somit ein gegenüber dem Stand der Technik höhere Wirkstoffkonzentration vor.

Diese wiederum begünstigt die oben ausgeführte Wirkung als Anti-Cellulitis-Mittel.

Zusammen mit dieser Wirkung auf Adipocyten geht ebenfalls einher, dass sich mit dem Pistia stratiotes Extrakt auch eine Behandlung von Akne vornehmen lässt, was in Eigenstudien ebenfalls nachgewiesen wurde. Die Extrakte reduzieren subcutane Hautfette, wodurch eine Besserung der Akne oder auch vorhanderer Abszesse erwirkt worden ist, ohne dass die Eiteransammlungen durch Öffnung oder Spaltung der Abszesse entfernt werden mussten, was immer auch eine Reinfektionsgefahr haben würde. Die Akne sowie die Abszesse trockneten förmlich aus.

### Regenerative und revitalisierende Aktivität der Haut:

### Zellwachstumseffekte.

Hierzu wurden humane Fibroplasten in einem Nährstoffmedium unter Zugabe von Pistia stratiotes Extrakt beobachtet.

Diese Studie aus "Journal Francais Hydrologie, 1991, 9, 149-156) zeigt dass Extrakte aus Pistia stratiotes das Wachstum und den Metabolismus der humanen Fibroplasten erheblich stimuliert.

### Überlebensfähigkeit

Tests ebenfalls an humanen Fibroplasten in Verbindung mit der Gabe von Pistia stratiotes Extrakten haben auch in Bezug auf die Überlebensfähigkeit von Zellen positive Ergebnisse geliefert. Das sogenannte Glutathion wird von Zellen produziert, um direkt gegen oxydativen Stress und Umwelteinflüssen wie hoher Schwermetallbelastung reagieren zu können. Ein erhöhter Anteil an reduziertem Glutathion nach Behandlung der Zellen mit den besagten Extrakten ist demnach ein Maß für die gesteigerte Überlebensfähigkeit der Zelle bei äußeren Stress und Belastungen.

Auch dieser Test wurde mehrfach verifiziert.

Pistia stratiotes Extrakt erhöht den Gehalt an Proteinen in humanen Fibroploasten.

Die Extrakte zeigen daher eine regenerierende und revitalisierende Aktivität an humanen Fibroplasten. Damit sind sie geeignet zum Einsatz in Anti-Ageing-Mitteln.

Bei all den genannten Untersuchungen liegt eine Erhöhung der gewünschten Wirkung mit der Höhe der Konzentration der genannten Pflanzenextrakte vor.

### Antibakterielle Wirkungen, Antibiotische Wirkungen

Aus dem International Research Journal of Pharmacy, Khan Md Ahad Ali et al, IRJP 2(2), 2011, 82-92, Cytotoxicity, antimicrobial and neuropharmacological evaluation of ethanolic extract of pistia stratiotes L., sind Beobachtungen bekannt, bei denen morphinartige Verbindungen im Pistia stratiotes Extrakt vermutet werden, um die entsprechende schmerzstillende Wirkung zu plausibilisieren. Die schmerzhemmende Wirkung wird dabei an phenolischen Verbindungen festgemacht.

Diese Zusammenhänge wurden dabei für ethanolische Auszüge aus Pistia stratiotes gefunden.

Gemäß der vorliegenden Erfindung, werden aber zunächst ausschließlich Kochauszüge, ohne externe Zugabe von destilliertem Wasser, also allein auf dem Zellwasserbeitrag der Frischmasse aus Pistia stratiotes vorgenommen. Damit ist der Auszug konzentrierter und wegen der Erhitzung in einem geschlossenen System, d.h einem Autoklaven bei niedrigerer Temperatur, vorzugsweise unterhalb von 80 °C, erheblich schonender gewonnen, und Oxidationsvorgänge weitestgehend unterbunden.

Vorzugsweise kann nach Befüllung des Autoklaven die Luft durch CO₂ oder N₂ oder einem Inertgas bzw einem anderen Inertgas oder Schutzgas verdrängt werden. Damit werden Oxidationvorgänge während der Erhitzung weitestgehend ausgeschlossen, und der Auszug findet sozusagen unter einer Schutzgasatmosphäre statt.

Auch bei dieser Anwendung spielen daher die erfindungsgemäßen Merkmale eine Rolle, die zu einem höher konzentrierten Extrakt führen, weil die Wirksamkeit mit der Konzentration steigt.

Dies ist jedoch nicht trivial, weil es sehr wohl auch hätte möglich sein können, dass eine höhere Konzentration der Wirkstoffe in den Extrakten auch schon hätte toxisch reagieren können.

In Bezug auf weitere aquatische Pflanzen, liegen ebefalls Nachweise zur

### Antibakterielle Wirkung, antibiotischen Wirkung

### vor.

So ist aus dem Aufsatz aus Ilhami Gülcin et al, Turkish Journal of Biology, 34(2010), Antioxidant, antibacteriial, and anticandidal activitis of an aquatic plant (Lemna minor L) bekannt, dass die Auswirkungen des Fungizids Folpet auf die enzymatische Abwehr gegen oxidativen Stress in den Sprossgliedern der Wasserlinsen untersucht wurden. Dabei wurde gezeigt, dass sie immunomodulatorische Fähigkeiten besitzt, d.h. die Fähigkeit die Phagozytose zu verbessern. Weiterhin wurden die kupferinduzierten Veränderungen in der Wirksamkeit von antioxidativ wirkenden Enzymen in den Sprossgliedern der Wasserlinse vielfach als Rohmaterial für die Herstellung von schmerzstillenden und fiebersenkenden Mitteln genutzt.

Auch sind Antioxidantien wie Karotenoide und Flavonoide und andere Phenolverbindungen daraus bekannt.

Weiterhin zeigt eine Gegenüberstellung mehrer aquatischer Pflanzen, die Inhaltsstoffspektren auch im vergleich zueinander, wie sie gezeigt sind in "Anjana Dewanji, Amino Acid Composition of Leaf Proteins extracted from some aquatic weeds, J. Agric. Food Chem 1993, 41, 1232-1236."

Aufgeführt sind dort
- Alternanthera philoxeroides
- Azolla pinnata
- Lemna minor
- Limnanthemum cristatum
- Pistia stratiotes

| | A.Phil | A.Pinnata | Lemna | L.cristatum | Pistia s. | |
|---|---|---|---|---|---|---|
| Stickstoff % | 7,7 | 6,3 | 6,1 | 8,7 | 8,2 | |
| Rohfett % | 11,0 | 9,9 | 11,4 | 8,4 | 14,4 | |
| Rohfaser % | 4,6 | 2,8 | 2,7 | 3,4 | 1,5 | |
| Asche % | 7,3 | 4,1 | 6,0 | 4,4 | 5,8 | |
| Kaloriewert kcal/g | 2,5 | 4,2 | 2,8 | 4,3 | 3,6 | |
| β-carotine *µ*g/g | 462,5 | 632,8 | 627,2 | 674,7 | 653,7 | |
| Polyphenole gesamt % | 2,9 | 1,7 | 2,1 | 2,7 | 1,3 | |
| In vitro-Verwertbarkeit % | 71,5 | 77,7 | 77,9 | 78,1 | 80,7 | |

Daraus ersichtlich ist die durchweg ausgewogene Balance der Inhaltsstoffe.

Auch diese bekannten Analysen beziehen sich auf Auszugsverfahren mit der Zugabe von Wasser.

Davon grenzt sich das erfindungsgemäße Auszugsverfahren dadurch ab, dass ohne Zugabe von zusätzlichem Wasser, allein auf der Basis von Frischmasse erhitzt wird in einem geschlossen System, d.h. einem Autoklaven.

Da aquatische Pflanzen in der Mittel ohnehin nur einen Trockensubstanzgehalt von ca 10% aufweisen, kommt so genügend Zellwasser mit in den Prozess, so dass beim Erhitzen dieses quasi als intrinsisches Auszugsmedium zur Verfügung steht.

Die Verwendung eines Autoklaven, der dann nach Befüllen vorzugsweise auch noch die Luft verdrängend mit einem Inertgas wie Stickstoff oder auch ein anderes Inertgas gefüllt und verschlossen wird, lässt einen äußerst schonenen Auszug entstehen.

In Bezug auf die genannten Inhaltsstoffe ist dieses erfindungsgemäße Auszugsverfahren erheblich schonender. Selbes gilt in Bezug auf Proteine.

In der nachfolgenden Darstellung ist der wesentliche Aminosäureaufschluss inhaltsstofflich dargestellt.

| | A.Phil | A.Pinnata | Lemna | L.cristatum | Pistia s. | |
|---|---|---|---|---|---|---|
| Lysin | 6,93 | 6,1 | 5,93 | 4,64 | 7,04 | |
| Histidin | 2,62 | 2,27 | 2,65 | 2,14 | 2,88 | |
| Arginin | 5,47 | 6,22 | 5,99 | 5,98 | 6,31 | |
| Asparetinsaure | 10,2 | 10,34 | 10,56 | 10,14 | 9,62 | |
| Threonin | 4,91 | 4,97 | 5,13 | 4,88 | 4,76 | |
| Serin | 4,97 | 5,32 | 5,54 | 4,86 | 4,84 | |
| Glutaminsäure | 14,01 | 13,83 | 13,6 | 14,37 | 13,44 | |
| Prolin | 5,78 | 4,73 | 4,53 | 5,59 | 5,04 | |
| Glycin | 5,55 | 5,76 | 5,62 | 5,83 | 5,74 | |
| Alanin | 6,13 | 7,00 | 7,11 | 6,43 | 6,34 | |
| Valin | 6,77 | 6,76 | 6,43 | 7,01 | 6,73 | |
| Methionin | 1,24 | 1,22 | 1,38 | 1,43 | 1,10 | |
| Isoleucin | 6,01 | 5,95 | 5,67 | 5,99 | 5,96 | |
| Leucin | 9,39 | 9,44 | 9,6 | 9,82 | 9,62 | |
| Tyrosin | 4,15 | 4,17 | 4,24 | 4,57 | 4,6 | |
| Phenylalanin | 5,86 | 5,92 | 6,01 | 6,31 | 5,96 | |
| Rohprotein | 48,2 | 39,1 | 38,3 | 54,2 | 51,10 | |

Die Gegenüberstellung der Proteine d.h. des Aminosäureaufschlusses im Detail zeigt, dass die vertretenen Kandidaten über viele aquatische Pflanzen hinweg jeweils zumindest in der selben Größenordnung liegen.

Teil der Erfindung ist auch die Schaffung verschiedener Produktsysteme.

Die Zeichnungen zeigen Ausführungsbeispiele bezüglich des Verfahrensablaufes.

Es zeigt:
- Figur 1:: Verfahrensablauf bei der Herstellung einer Salbe oder Creme
- Figur 2:: Herstellung des Extraktes selbst, respektive weiterer Anwendungen.

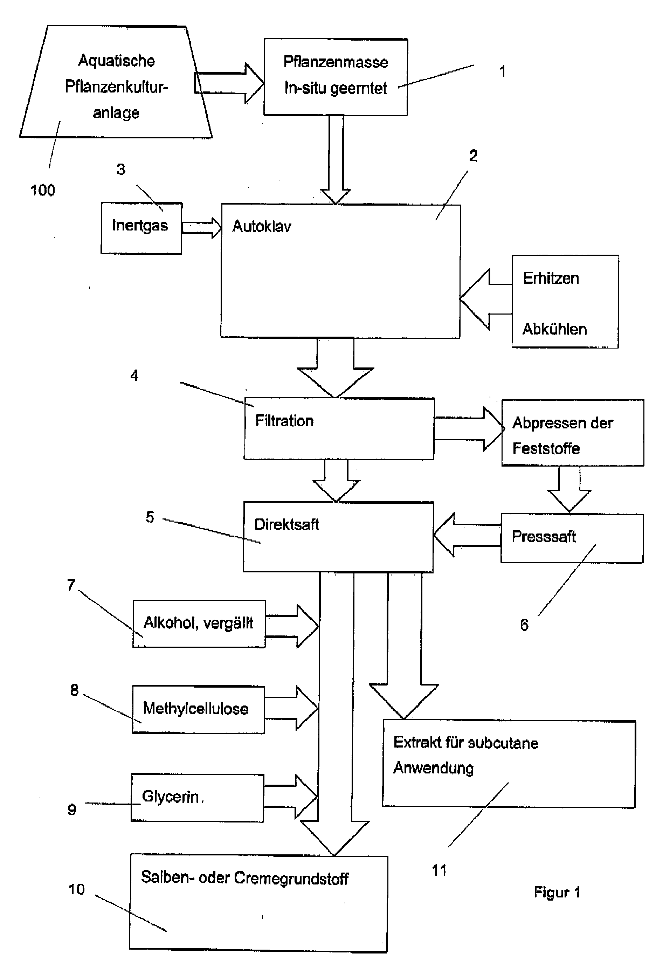

Figur 1 zeigt den verfahrensablauf mit den jeweils wichtigen Verfahrensschritten in chronologischer Reihenfolge.

Aus einer Kulturanlage 100 für aquatische Pflanzen werden in diesem Ausführungsbeispiel Pflanzen in- situ geerntet. Die Kulturanlagen beinhalten patentierte Stapelsysteme für aquatische Pflanzen, bei denen Teilmengen des Pflanzenbesatzes täglich oder wöchentlich geerntet werden, und somit als Frischmasse hoch verfügbar sind. Es ist aber auch möglich, die Ernte direkt auf die Produktionszyklen anzupassen. Die aquatischen Pflanzen laufen bei ihrer selbsttätigen Population in Grenzbedeckungen, die automatisch eine Supression des weiterwuches bewirken. Die Kultur selbst bleibt in diesem Zustand regenerativ selbsterhaltend und somit stets frisch und nicht überaltert.

Anders als bei jeder anderen Biomasse kann aufgrund der extrem schnellwüchsigen Population von aquatischen Pflanzen, erst diese hohe Verfügbarkeit genutzt und in den Produktionsprozess logistisch integriert werden. Die geerntete Pflanzenmasse wird als direkte Frischmasse, oder als vakuumverpackte Frischmasse einem Autoklaven zugeführt. Blätter und übrige Pflanzenteile werden dazu unmittelbar vor dem Zuführen in den Autoklaven zerkleinert. Sodann kann vorzugsweise der befüllte Autoklav 2 mit Inertgas gespült werden um Luft und Luft-Sauerstoff auszublasen und mit einem Inertgas zu spülen, Beispielsweise N₂.

Sodann wird der Autoklav mit Inhalt erhitzt für eine Zeit zwischen 10 bis 60 Minuten bei einer Temperatur kleiner der gleich 100 °C, vorzugsweise bei 80°C.

Der Autoklav bleibt bis zum Abkühlen geschlossen, damit die in dem Kondensat aufgenommenen ätherischen Substanzen im Autoklaven verbleiben und zurücktropfen. Gegebenenfalls wird der Autoklav mechanisch oder mit Schallwellen beaufschlagt, um die Rücktropfung des Kondensates zu bewirken.

Nach dem Abkühlen wird der Inhalt des Autoklaven 2 in eine Filtration 4 gegeben. Der bei Filtration ablaufende Saft (Direktsaft 5) wird aufgefangen. Sodann werden die in der Filtration verbliebenen Feststoffe nochmals gepresst und der Presssaft 6 entweder dem Direktsaft 5 mit zugeführt oder als höher konzentrierter Saft gesondert weiter verarbeitet.

Sodann wird bspw das Gemisch aus Direktsaft 5 und Pressaft 6, welche wahlweise zusammengeführt wurden nun zunächst mit reinem Alkohol 7, vorzugsweise in einer Mengenzugabe von 25 % des Volumens des Saftes -Presssaft, bzw Presssaft und Direktsaft-Gemisches zugeführt und vermischt wird.

Hernach wird diesem so erhaltenen Gemisch Methylcellulose und hernach Glycerin zugesetzt. Wahlweise kann anstatt der Zugabe von Methylcellulose und/oder Glycerin auch eine Cremegrundbasis zugeführt werden.

Nach entsprechender guter Vermischung wird somit ein Grundstoff für weitere Produkte geschaffen wie sie bereits oben erwähnt sind.

Wahlweise ist es jedoch auch möglich, wie Figur 1 zeigt, den Direktsaft oder den Presssaft oder die Mischung aus Direktsaft und Pressaft direkt, d.h. nach einer weiteren Filtration und einem Hygienisierungschritt als Extrakt für eine subcutane Anwendung bereit zustellen.

Der Hygienisierungsschritt kann bspw in einer weiteren Erhitzung des Saftes vorgenommen werden. Vorzugsweise wieder unter einer Inertgasatmosphäre.

Die übliche Anwendung des so erhaltenene Grundstoffes oder einer Salbe oder Creme ist, dass diese auf die Haut aufgebacht wird, und zwar auf befallene Hautpartien. Für die Anwendung als Anticellulitis Präparat kann diese Creme oder Salbe auch auf die Haut aufgebracht werden. Wegen der hohen Diffusivität zieht diese extrem schnell ein, und diffundiert auch in die befallenen subcutanen Regionen.

Zur Verstärkung des Effektes kann aber auch eine subcutane Einbringung des Extraktes in die befallene Haut- oder Körperregionen durch eine Spritze, oder ein Nadelpad oder eine Nadelrolle eingebracht werden.

In Bezug auf pharmazeutische Produkte, sind mit diesem Herstell-Verfahren alle in den oben genannten Studien erwähnten Anwendungen denkbar.

Von Präparaten zur Anticellulitis Behandlung bis hin zu schmerzstillenden Anwendungen, bis hin zur Gabe des Extraktes nach einem der Ansprüche 1 bis 5 in Form eines Sprayapplikators zur Behandlung von entzündlichem Schnupfen oder Allergien, wozu eine schwache Salzlösung mit eingemischt würde.

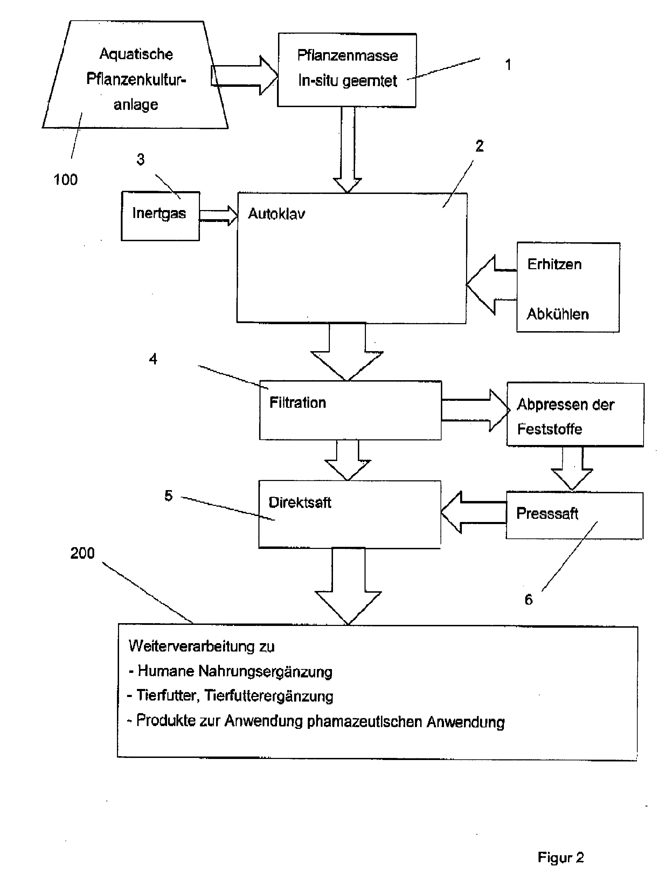

Figur 2 zeigt den Verfahrenslauf nur bis zum Erhalt des eigentlichen Extraktes, der sodann unter anderem für die subcutane Anwendung oder aber auch für Nahrungsergänzungsprodukte, oder Produkte für die Tierernährung, oder für pharmazeutische Anwendungen weiter verwendet wid.

### Bezugszeichen:

- 1: aquatische Pflanzenmasse, frisch
- 2: Autoklav
- 3: Inertgaszugabe
- 4: Filtration
- 5: Direktsaft
- 6: Presssaft
- 7: Alkoholzugabe
- 8: Methylcellulose
- 9: Glycerin
- 10: Salben und Cremegrundstoff
- 11: Extrakt für subcutane Anwendung

- 100: Aquatische Kulturanlage
- 200: Direkte Weiterverarbeitung des Extraktes

## Patentansprüche

1. Verfahren zur Herstellung eines Pflanzenextraktes aus aquatischen Pflanzen, bei dem das Pflanzenmaterial nach der Ernte über einen Schritt der Dünstung wärmebehandelt wird,
**dadurch gekennzeichnet,**
**dass** die geernteten aquatischen Pflanzen in ihrer Frischmasse ohne Zugabe von Wasser in einem Autoklaven geschlossen erhitzt werden.

2. Verfahren nach Anspruch 1,
**dadurch gekennzeichnet,**
**dass** die aquatischen Pflanzen oder Teile davon zuvor gewaschen und zerkleinert werden, und dass sie in dem Autoklaven für eine Dauer von 10 bis 60 Minuten bei einer Temperatur T ≤ 100°C erhitzt werden.

3. Verfahren nach Anspruch 1 oder 2,
**dadurch gekennzeichnet,**
**dass** nach Befüllung des Autoklaven, die Luft aus demselben durch Zugabe eines Inertgases verdrängt wird.

4. Verfahren nach Anspruch 2 oder 3,
**dadurch gekennzeichnet,**
**dass** der Autoklav samt Inhalt im geschlossenen Zustand abgekühlt wird, und ein Rücktropfvorgang das Kondenswasser samt darin enthaltener ätherischer Bestandteile in den so erhaltenen Sud mit einbringen.

5. Verfahren nach Anspruch 4,
**dadurch gekennzeichnet,**
**dass** der Sud filtiert, und die rückgehaltene Festmasse ausgepresst wird, derart dass Sud und Presswasser zusammengeführt, und die festen Bestandteile aus der weiteren Bearbeitung abgetrennt werden.

6. Verfahren nach Anspruch 5,
**dadurch gekennzeichnet,**
**dass** dem Sud in einer Menge von ca. 25 Volumenprozent des Sudvolumens Alkohol zugegeben und damit vermischt wird.

7. Verfahren nach Anspruch 6,
**dadurch gekennzeichnet,**
**dass** der Alkohol vergällt ist.

8. verfahren nach einem der vorhergehenden Ansprüche, **dadurch gekennzeichnet,**
**dass** dem Gemisch nun Methylcellulose und/oder eine Salben- oder Cremebasis zugeführt wird.

9. Verfahren nach Anspruch 8,
**dadurch gekennzeichnet,**
**dass** dem Gemisch Glycerin zugegeben wird, zur Vermeidung einer Schmierung oder Schuppung auf der Haut.

10. Verfahren nach einem der vorhergehenden Ansprüche,
**dadurch gekennzeichnet,**
**dass** die aquatischen Pflanzen zur Gewinnung von Resveratrol herangezogen werden.

11. Verfahren nach einem der vorhergehenden Ansprüche,
**dadurch gekennzeichnet,**
**dass** als aquatische Pflanzen
Pistia stratiotes und/oder
Lemnaceae und/oder
Alternanthera philoxeroides und/oder
Eichhornia crassipes und/oder
Azolla und/oder
Limnathemum cristatium
Spirodela
eingesetzt werden.

12. Verfahren nach einem der Ansprüche 1 bis 11,
**dadurch gekennzeichnet,**
**dass** die genannten Pflanzen, oder die Blätter der genannten Pflanzen oder die zerkleinerten Teile der genannten Pflanzen vor der Erhitzung mit Natriumhydrogencarbonat bestäubt werden.

13. Grundstoff für dermatologische oder pharmazeutische Anwendung, hergestellt nach dem Verfahren nach einem der Ansprüche 1 bis 12 für Salben oder Cremes zur Behandlung von Akne, und/oder Neurodermitis, und/oder Cellulitis und/oder Fettabbau im subcutanen Gewebe.

14. Grundstoff für pharmazeutische Anwendung, hergestellt zumindest nach einem der Ansprüche 1 bis 5.

15. Grundstoff nach einem der Ansprüche 1 bis 5, zur Herstellung eines humanen Nahrungsergänzungsmittels.

16. Grundstoff nach einem der Ansprüche 1 bis 5, zur Herstellung eines Tierfuttermittels oder Tierfutterergänzungsmittels.

17. Verfahren zur Gabe der Wirkstoffe oder des Grundstoffes nach Anspruch 13 oder 14,
**dadurch gekennzeichnet,**
**dass** zusätzlich oder anstatt der von außen auf die Haut verabreichte Gabe der Wirkstoffe oder des Grundstoffes aus aquatischen Pflanzen, vorzugsweise aus der Pistia stratiotes, dieselbe unter die Oberhaut injiziert wird, durch einzelne Injektionsnadeln oder eine Mehrfachanordung von Injektionsnadeln auf einem Stempel oder einer Walze.

18. Verfahren zur sortenreinen Kultivierung von Pistia stratiotis, und/oder Eichhornia crassipes, und/oder anderen aquatischen Pflanzen zur Bereitstellung von Pflanzenmaterial für das Verfahren nach einem der Ansprüche 1 bis 12,
**dadurch gekennzeichnet,**
**dass** die Pistia stratiotis oder die Eichhornia, oder unter reduziertem Tageslicht bei einer Beleuchtungsstärke von kleiner oder gleich ca 50% der natürlichen Beleuchtungsstärke der jeweiligen Region liegt, in der sie kultiviert wird, derart, dass die sexuelle Vemehrung der Pistia stratiotis unterdrückt und die vegetative Vermehrung so begünstigt wird.

19. verfahren nach Anspruch 18,
**dadurch gekennzeichnet,**
**dass** in das Kulturwasser DL-Milchsäure, und/oder Vitamine, insbesondere Vitamin B12, und/oder Thio-Milchsäure, und/oder
Kaliumhydrogencarbonat, und/oder
Ascorbinsäure, und/oder
Methanol, und/oder
NPK-Dünger, und/oder
feinstgemahlenes Steinmehl, und/oder Spurenelemente,
zugegeben wird bzw. werden.

20. Verfahren nach Anspruch 19
**dadurch gekennzeichnet;**
**dass** unmittelbar vor Gabe der genannten zugegebenen Stoffe die aquatischen Pflanzen einen Wurzelschnitt erhalten.
